# EUROPÄISCHE PATENTSCHRIFT

(11) **EP 3 747 348 B1**
(45) Veröffentlichungstag und Bekanntmachung des Hinweises auf die Patenterteilung: **07.10.2026**
(21) Anmeldenummer: 20175771.3
(22) Anmeldetag: 20.05.2020
(51) Int. Cl.: A61B 1/04, A61B 1/06, A61B 5/00, G01J 3/10, G01J 3/44, G01N 21/64, G02B 23/24, A61B 1/00

(54) **LICHTQUELLE UND SYSTEM FÜR DIE FLUORESZENZDIAGNOSE**
LIGHT SOURCE AND SYSTEM FOR FLUORESCENCE DIAGNOSIS
SOURCE LUMINEUSE ET SYSTÈME DE DIAGNOSTIC DE FLUORESCENCE

(30) Priorität: 06.06.2019 DE 102019115276
(43) Veröffentlichungstag der Anmeldung: 09.12.2020
(60) Teilanmeldung: 26183017.8 / 4 778 443
(73) Patentinhaber: Karl Storz SE & Co. KG, 78532 Tuttlingen (DE)
(72) Erfinder: Göbel, Werner, 78532 Tuttlingen (DE); Spath, Henrik, 78532 Tuttlingen (DE); Bauer, Franz, 78532 Tuttlingen (DE); Pascale, Costantino, 78532 Tuttlingen (DE)
(74) Vertreter: Witte, Weller & Partner Patentanwälte mbB

(56) Entgegenhaltungen:
- DE-A1- 10 252 313
- US-A1- 2016 062 103

## Beschreibung

Die Erfindung betrifft eine Lichtquelle für die Fluoreszenzdiagnose, ein System für die Fluoreszenzdiagnose mit einer solchen Lichtquelle, sowie ein Verfahren zur Fluoreszenzdiagnose.

Die Fluoreszenzdiagnose wird in der Medizin zur Beurteilung des Zustands von biologischem Gewebe, beispielsweise allgemein zur Gewebedifferenzierung, insbesondere zur Tumorerkennung, aber auch zur Erkennung der Durchblutung und Vitalität verwendet. Die Fluoreszenzdiagnose kann aber auch für technische Diagnosezwecke in industriellen oder wissenschaftlichen Anwendungen verwendet werden. Ohne Beschränkung der Allgemeinheit wird die Erfindung anhand der medizinischen Fluoreszenzdiagnose beschrieben.

Im medizinischen Bereich hat sich die Fluoreszenzdiagnose zu einer vielversprechenden Alternative bzw. Ergänzung in der Erkennung und Behandlung z.B. neoplastischer Veränderungen entwickelt. Die Fluoreszenzdiagnose beruht auf der Wechselwirkung von Licht einer geeigneten Wellenlänge mit einer im zu untersuchenden Gewebeareal vorhandenen fluoreszierenden Substanz. Dabei kann eine fluoreszierende Substanz ein Fluoreszenzfarbstoff sein, der zuvor in das zu untersuchende Gewebeareal eingebracht wurde, beispielsweise durch Verabreichen der fluoreszierenden Substanz selbst oder einer Vorstufe derselben an den zu untersuchenden Patienten. Eine fluoreszierende Substanz kann jedoch auch eine bereits im Zielgebiet vorhandene Substanz sein, beispielsweise eine gewebespezifische Substanz, die durch Licht in einem geeigneten Spektralbereich zur Autofluoreszenz angeregt wird. Die vorliegende Erfindung kann beide Fälle umfassen.

Bei der Fluoreszenzdiagnose wird üblicherweise nicht nur die Fluoreszenz detektiert, sondern es wird bei der Fluoreszenzbeobachtung zusätzlich der Hintergrund, d.h. Bereiche im Bild des Beobachtungsareals ohne Fluoreszenz, ausgeleuchtet, damit sich der Anwender des Systems, üblicherweise ein Arzt, besser im Beobachtungsareal orientieren kann und auch die Gewebedifferenzierung verbessert wird.

Herkömmliche Fluoreszenzdiagnosesysteme weisen eine Lichtquelle mit einer Xenonlampe auf. Bei solchen Xenonlampen-basierten Systemen wird eine Resttransmission des Anregungslichts zugelassen, beispielsweise aus einem überlappenden Bereich des in der Lichtquelle verbauten Anregungs- mit dem Beobachtungsfilter oder mittels erhöhter Transmission innerhalb des Detektionsbereichs des Beobachtungsfilters. Erforderlich ist dabei, dass das detektierte Hintergrundlicht in einem vom Fluoreszenzsignal separaten Kamerakanal detektiert wird. Liegt beispielsweise die Fluoreszenz im nah-infraroten Spektralbereich und wird diese im Blau-Kanal der Kamera detektiert, so ist das Licht für die Hintergrundausleuchtung im grün-roten Spektralbereich zur Verfügung zu stellen, um eine eindeutige Zuordnung von Fluoreszenz und Hintergrund zu gewährleisten.

Xenonlampen-basierte Lichtquellen haben den Nachteil einer relativ kurzen Lebensdauer. Der Nachteil solcher herkömmlicher Fluoreszenzdiagnosesysteme besteht daher zum einen in dem durch den Austausch der Xenonlampe bedingten Kostenmehraufwand und zum anderen in den Stillstandzeiten, die durch die Wartung bzw. den Austausch bedingt sind.

Es sind, beispielsweise aus DE 10 2013 111 368 A1, inzwischen Lichtquellen für die Fluoreszenzdiagnose bekannt geworden, die Halbleiterleuchtmittel, wie Leuchtdioden, Laserdioden, Superlumineszenzdioden und dergleichen aufweisen. Auch bei Halbleiterleuchtmittel-basierten Lichtquellen für die Fluoreszenzdiagnose sollten wie bei den herkömmlichen Xenonlampen-basierten Lichtquellen Beleuchtungsmodi für Weißlicht-Beobachtung und Fluoreszenzanregung zur Verfügung gestellt werden, wobei im Fluoreszenzmodus wiederum eine Ausleuchtung des Bildhintergrunds, d.h. der Bildbereiche ohne Fluoreszenz, gewünscht ist, um dem Anwender die Möglichkeit der Detektion beispielsweise bestimmter anatomischer Strukturen zur besseren Orientierung zu geben.

Halbleiterleuchtmittel-basierte Lichtquellen für die Fluoreszenzdiagnose weisen beispielsweise eine breitbandig emittierende Leuchtdiode, insbesondere eine Weißlicht-Leuchtdiode, sowie eine weitere, schmalbandig emittierende Leuchtdiode auf, wobei Letztere im Spektralbereich der Absorption des Fluoreszenzfarbstoffs emittiert. Durch diesen Ansatz ist ein schnelles, ggf. Kamera-synchrones Umschalten zwischen Weißlicht und Fluoreszenzanregungslicht sowie die simultane Weißlicht-/Fluoreszenzbeleuchtung (im letzteren Fall für Zwei-Chip-Kameras) möglich. Für ein andauerndes Arbeiten im Fluoreszenzmodus ist jedoch wie bei bisherigen Xenonlampen-basierten Lichtquellen auch eine Hintergrundausleuchtung vorteilhaft. Im Falle beispielsweise einer Phosphor-konvertierten Weißlicht-Leuchtdiode würde das Hinzuschalten dieser Leuchtdiode jedoch den gesamten sichtbaren Spektralbereich und damit alle Kamerakanäle (rot-grün-blau) überdecken, d.h. auch den Kamerakanal, der die Fluoreszenz detektiert. Die Hintergrundausleuchtung muss daher innerhalb des Spektralbereichs des Kamerakanals zur Verfügung gestellt werden, der kein Fluoreszenzsignal detektiert.
US 2016/0062103 A1 offenbart ein Endoskopiesystem und ein Verfahren zum Betreiben eines Endoskopiesystems, das ein gleichzeitiges Beleuchten eines Gewebes mit NIR-Anregungslicht und sichtbarem Licht, Abbilden des Gewebes unter Verwendung eines einzigen Detektors und unabhängiges Einstellen einer Intensität des NIR-Anregungslichts und einer Intensität des sichtbaren Lichts aufweist.
DE 102 52 313 A1 offenbart ein Untersuchungssystem und ein Untersuchungsverfahren, um in einem Gewebebereich eine Fluoreszenzmarkierung und eine Umgebung in derselben sichtbar zu machen. Das Verfahren umfasst Beleuchten der Gewebeumgebung mit Licht aus einem ersten Wellenlängenbereich, der ein Anregungsspektrum der Fluoreszenzmarkierung umfasst, wobei die Fluoreszenzmarkierung in einem Fluoreszenzspektrum emittiert, und Beleuchten des Gewebebereichs mit Licht aus wenigstens einem zweiten Wellenlängenbereich, der das Fluoreszenzspektrum im Wesentlichen nicht umfasst und der einen Teilwellenlängenbereich umfasst, welcher den Anregungswellenlängenbereich nicht umfasst, wobei eine Intensität der Beleuchtungslichtquellenanordnung relativ zu einer Intensität der Anregungslichtquellenanordnung geändert werden kann.

Der Erfindung liegt die Aufgabe zugrunde, eine Lichtquelle für die Fluoreszenzdiagnose bereitzustellen, die auf Halbleiterleuchtmitteln basiert und im Fluoreszenzmodus eine Hintergrundausleuchtung zur verbesserten Orientierung im Beobachtungsareal ohne Überstrahlen der Fluoreszenz ermöglicht.

Der Erfindung liegt des Weiteren die Aufgabe zugrunde, ein verbessertes System für die Fluoreszenzdiagnose bereitzustellen.

Erfindungsgemäß wird eine Lichtquelle für die Fluoreszenzdiagnose bereitgestellt, wobei die Lichtquelle einen Beleuchtungsstrahlengang bereitstellt, mit einer ersten Halbleiterleuchtmittel-basierten Lichtemissionseinheit, die dazu ausgelegt ist, erstes Licht in einem breitbandigen ersten Wellenlängenspektrum zu emittieren, und mit einer zweiten Halbleiterleuchtmittel-basierten Lichtemissionseinheit, die dazu ausgelegt ist, zweites Licht in einem schmalbandigen zweiten Wellenlängenspektrum zur Anregung einer Fluoreszenz zu emittieren, weiterhin mit einem Spektralfilter für die erste Lichtemissionseinheit, das dazu ausgelegt ist, Spektralanteile des ersten Wellenlängenspektrums, die einem Farbkanal einer Kamera zugeordnet sind, mit dem die Fluoreszenz zu detektieren ist, zu blockieren, und übrige Spektralanteile des ersten Wellenlängenspektrums durchzulassen, weiterhin mit einer Helligkeitssteuerung für die erste Lichtemissionseinheit zum Dimmen der Intensität des emittierten ersten Lichtes, und mit einem optischen Intensitätsabschwächungselement für die erste Lichtemissionseinheit, mit dem die Intensität des emittierten ersten Lichtes unter die mittels der Helligkeitssteuerung erreichbaren minimalen Intensität reduziert werden kann, wobei das Intensitätsabschwächungselement beweglich ist, um in dem Beleuchtungsstrahlengang der ersten Lichtemissionseinheit eingebracht zu werden und aus diesem entfernt zu werden, wobei das Spektralfilter und das Intensitätsabschwächungselement an einem gemeinsamen Filterträger in Richtung des Beleuchtungsstrahlengangs hintereinander angeordnet sind, wobei der Filterträger einen Stellantrieb aufweist, wobei der Stellantrieb mit einer Steuereinrichtung verbunden ist, die beim Umschalten der Lichtquelle zwischen Weißlichtmodus und Fluoreszenzmodus den Stellantrieb aktiviert, um das Spektralfilter und das Intensitätsabschwächungselement im Weißlichtmodus aus dem Beleuchtungsstrahlengang der ersten Lichtemissionseinheit zu entfernen und im Fluoreszenzmodus in diesen einzubringen.

Die erste Halbleiterleuchtmittel-basierte Lichtemissionseinheit emittiert Licht in einem breitbandigen ersten Wellenlängenspektrum, das für eine Weißlichtbeobachtung des Beobachtungsareals vorzugsweise unverändert in das Beobachtungsareal übertragen wird, und das im Fluoreszenzbeobachtungsmodus (kurz Fluoreszenzmodus) in bestimmter Weise spektral und intensitätsmäßig verändert wird, um für eine geeignete Hintergrundausleuchtung zu sorgen. Für die spektrale Veränderung des von der ersten Lichtemissionseinheit emittierten ersten Lichtes zur Bereitstellung einer Hintergrundausleuchtung des Beobachtungsareals weist die Lichtquelle ein Spektralfilter auf, das im Fluoreszenzmodus in den Beleuchtungsstrahlengang der ersten Lichtemissionseinheit eingebracht werden kann. Dieses Spektralfilter ist bezüglich der spektralen Transmissionscharakteristik dahingehend ausgelegt, Spektralanteile des ersten Lichtes zu blocken, die demjenigen Kamerakanal zugeordnet sind, mit dem die Fluoreszenz detektiert werden soll, und gleichzeitig die übrigen Spektralanteile des ersten Lichtes, die den Spektralbereichen der verbleibenden Kamerakanäle entsprechen, zu transmittieren. Erfindungsgemäß ist jedoch nicht nur eine spektrale Veränderung des von der ersten Lichtemissionseinheit emittierten Lichtes vorgesehen, sondern auch eine Veränderung der Intensität des von der ersten Lichtemissionseinheit emittierten Lichtes zum Zwecke der Hintergrundausleuchtung. Hierzu ist eine Helligkeitssteuerung für die erste Lichtemissionseinheit vorgesehen, mit der die Intensität des von der ersten Lichtemissionseinheit emittierten ersten Lichtes gedimmt werden kann. Die Helligkeitssteuerung ist dazu ausgelegt, die Intensität des ersten Lichtes kontinuierlich zwischen zwei Intensitätswerten, beispielsweise einer maximalen und einer minimalen Intensität, zu steuern. Die Helligkeitssteuerung kann als elektrische oder elektronische Dimmschaltung ausgestaltet sein, die den Treiberstrom der ersten Lichtemissionseinheit steuert. In anderen Ausführungsformen kann die Helligkeitssteuerung auch eine kontinuierlich dimmende Dimmscheibe aufweisen, die mit einem motorischen Antrieb versehen ist.

Für die Hintergrundausleuchtung wird Licht von sehr schwacher Intensität benötigt, um die typischerweise schwachen Fluoreszenzsignale nicht zu überstrahlen. Wenn die Helligkeitssteuerung beispielsweise als elektrische Dimmschaltung ausgebildet ist, kann das Dimmen mittels der Helligkeitssteuerung für die erforderliche Verringerung der Intensität der Hintergrundausleuchtung nicht ausreichend sein, da Halbleiterleuchtmittel wie beispielsweise Leuchtdioden die Eigenschaft besitzen, einen Minimalstrom für einen zuverlässigen Betrieb zu erfordern. D.h. der Treiberstrom der Leuchtdiode kann nicht auf einen beliebigen Wert minimiert werden, sondern nur bis zu einer bestimmten Schwelle. Sobald diese Schwelle unterschritten ist, findet keine kontinuierliche Lichtemission der Leuchtdiode statt. Oberhalb dieser minimalen Bestromungsschwelle liefern Leuchtdioden zwar gedimmtes Licht, dessen Intensität allerdings liegt immer noch deutlich über den für Hintergrundausleuchtung benötigten Werten. Daher weist die erfindungsgemäße Lichtquelle weiterhin ein optisches Intensitätsabschwächungselement für die erste Lichtemissionseinheit auf, mit dem die Intensität des emittierten ersten Lichtes unter die mittels der Helligkeitssteuerung ohne das Intensitätsabschwächungselement erreichbare minimale Intensität reduziert werden kann.

Das optische Intensitätsabschwächungselement kann ein oder mehrere mechanische Gitter aufweisen, oder in anderen Ausführungsformen ein oder mehrere Neutraldichtefilter. Ein Neutraldichtefilter reduziert breitbandig die Intensität von Licht um einen bestimmten Faktor, der typischerweise 10- bis 1000-fach beträgt. Das Intensitätsabschwächungselement wird im Fluoreszenzmodus der Lichtquelle gleichzeitig mit dem Spektralfilter im Beleuchtungsstrahlengang der ersten Lichtemissionseinheit verwendet. Somit findet gleichzeitig eine spektrale wie auch eine intensitätsmäßige Anpassung des von der ersten Lichtemissionseinheit emittierten Lichtes für die Zwecke der Hintergrundausleuchtung statt.

Ein weiterer Vorteil der Kombination aus Helligkeitssteuerung und optischem Intensitätsabschwächungselement besteht darin, dass mittels der Helligkeitssteuerung eine Einstellung der Intensität der Hintergrundausleuchtung über einen kontinuierlichen Bereich von Intensitätswerten möglich ist, was wiederum durch Verwendung allein eines optischen Intensitätsabschwächungselementes, bspw. eines Neutraldichtefilters mit fester Neutraldichte nicht möglich ist. Das Neutraldichtefilter sorgt für eine starke Reduzierung der Intensität um beispielsweise über 90%, und die Helligkeitssteuerung ermöglicht zusätzlich dem Anwender eine Feineinstellung (Erhöhung oder Reduzierung) bzw. kontinuierliche variable Einstellung der Helligkeit der Hintergrundausleuchtung, wie sie für den Anwender und/oder die jeweilige Applikation (bspw. je nach diagnostischem Fachgebiet) am besten geeignet ist. Im Falle einer Helligkeitssteuerung in der Art einer Dimmschaltung, mit der der Treiberstrom der Halbleiter-basierten Lichtemissionseinheit so weit wie möglich reduziert werden kann, besteht ein weiterer Vorteil darin, dass die Verlustleistung und Wärmeabgabe der Lichtemissionseinheit minimiert werden kann.

Um die Lichtquelle sowohl für einen Weißlichtmodus als auch für einen Fluoreszenzmodus verwenden zu können, sind das Spektralfilter und das Intensitätsabschwächungselement beweglich, um in den Beleuchtungsstrahlengang der ersten Lichtemissionseinheit eingebracht bzw. aus diesem entfernt werden zu können.

Das Einbringen und Entfernen des Spektralfilters und des Intensitätsabschwächungselements ist automatisiert realisiert, und findet automatisch beim Umschalten der Lichtquelle zwischen Weißlichtmodus und Fluoreszenzmodus durch eine Steuerungseinrichtung oder -schaltung statt.

Das Spektralfilter und das Intensitätsabschwächungselement können translatorisch oder rotatorisch beweglich sein.

Das Spektralfilter und das Intensitätsabschwächungselement sind an einem gemeinsamen Träger in Richtung des Beleuchtungsstrahlengangs hintereinander angeordnet, wobei der Träger einen Stellantrieb aufweist.

Hierdurch wird eine besonders kompakte Ausgestaltung der Lichtquelle bereitgestellt, indem das Intensitätsabschwächungselement und das Spektralfilter eine optische Einheit bilden. Der genannte Stellantrieb des Filterträgers ist mit einer Steuerschaltung verbunden, die beim Umschalten der Lichtquelle zwischen Weißlichtmodus und Fluoreszenzmodus den Stellantrieb aktiviert, um das Spektralfilter und das Intensitätsabschwächungselement im Weißlichtmodus aus dem Beleuchtungsstrahlengang der ersten Lichtemissionseinheit zu entfernen, und im Fluoreszenzmodus in diesen einzubringen.

Die erste Lichtemissionseinheit weist vorzugsweise eine Weißlicht-Leuchtdiode auf. Die Weißlicht-Leuchtdiode kann eine Leuchtstoff-konvertierte, insbesondere Phosphor-konvertierte Weißlicht-Leuchtdiode sein. Das von der ersten Lichtemissionseinheit emittierte erste Licht weist somit ein Wellenlängenspektrum auf, das das gesamte sichtbare Spektrum umfassen kann.

Die zweite Lichtemissionseinheit kann eine schmalbandig emittierende Leuchtdiode oder eine Laserdiode aufweisen. Die schmalbandig emittierende Leuchtdiode oder die Laserdiode sind vorzugsweise auf das Absorptionsspektrum des verwendeten Fluoreszenzmarkers ausgelegt. Wenn beispielsweise als Fluoreszenzfarbstoff ICG (Indocyaningrün) verwendet wird, kann die zweite Lichtemissionseinheit beispielsweise eine schmalbandig emittierende Leuchtdiode oder Laserdiode mit einem Emissionspeak bei etwa 785 nm aufweisen. Für die Zwecke der Autofluoreszenzdiagnose kann die zweite Lichtemissionseinheit eine Leuchtdiode aufweisen, die einen Emissionspeak bei etwa 405 nm aufweist.

Vorzugsweise ist die Helligkeitssteuerung dazu ausgelegt, die Intensität des von der ersten Lichtemissionseinheit emittierten ersten Lichtes auf einen Wert im Bereich von 2% bis 15%, vorzugsweise von 5% bis 10% der (maximalen) Ausgangsintensität der ersten Lichtemissionseinheit zu reduzieren. Das optische Intensitätsabschwächungselement, das bspw. ein oder mehrere Neutraldichtefilter aufweist, ist wiederum vorzugsweise dazu ausgelegt, diese bereits durch die Helligkeitssteuerung reduzierte Lichtemission weiterhin zu reduzieren, beispielsweise um einen Faktor 10 bis 1000, vorzugsweise um einen Faktor 100 (bei einem Neutraldichtefilter entspricht dies der Dichte 2). Ziel ist es, die Intensität der Hintergrundausleuchtung in etwa an die Intensität der detektierten Fluoreszenzsignale anzupassen, so dass beides, Hintergrundausleuchtung und Fluoreszenzsignale in etwa ähnliche Intensitätswerte auf den jeweiligen Kamerasensoren (-chips)/ -kanälen erzeugen.

Eine derart stark reduzierte und an die Intensität der Fluoreszenzsignale angepasste Lichtintensität der Hintergrundausleuchtung ist für die Orientierung des Benutzers im Beobachtungsareal besonders gut geeignet. Eine solche Reduktion der Intensität des von der ersten Lichtemissionseinheit emittierten ersten Lichtes lässt sich durch Dimmen einer Leuchtdiode allein nicht erreichen. Daher ist die Kombination aus Helligkeitssteuerung und Intensitätsabschwächungselement, bspw. Neutraldichtefilter, besonders vorteilhaft.

Wenn das optische Intensitätsabschwächungselement ein Neutraldichtefilter aufweist, weist es vorzugsweise eine Neutraldichte in einem Bereich von 1 bis 4, vorzugsweise in einem Bereich von 1,5 bis 3, weiter vorzugsweise eine Neutraldichte von 2 auf.

Ein Neutraldichtefilter mit einer Neutraldichte von 1 besitzt eine Durchlässigkeit von 10%, d.h. ein Zehntel der auf das Filter einfallenden ursprünglichen Lichtmenge wird von dem Filter durchgelassen. Eine Neutraldichte von 2 bedeutet eine Durchlässigkeit von 1%, eine Neutraldichte von 3 eine Durchlässigkeit von 0,1%, und eine Neutraldichte von 4 eine Durchlässigkeit von 0,01%. Eine Neutraldichte von 2 ist besonders vorteilhaft in Kombination mit einer elektrischen Helligkeitssteuerung.

Das Spektralfilter kann ein Langpassfilter, ein Bandsperrfilter oder ein Kurzpassfilter sein. Ein Langpassfilter eignet sich, wenn die Fluoreszenz im Blaukanal detektiert wird, ein Bandsperrfilter, wenn die Fluoreszenz im Grünkanal detektiert wird, und ein Kurzpassfilter, wenn die Fluoreszenz im Rot-Kanal der Kamera detektiert wird.

Weiter wird erfindungsgemäß ein System für die Fluoreszenzdiagnose bereitgestellt, mit einer Lichtquelle nach einer oder mehreren der vorstehend genannten Ausgestaltungen, und mit einer Kamera mit einem ersten Farbkanal zur Detektion von Fluoreszenz in einem Beobachtungsareal und weiteren Farbkanälen zur Aufnahme von Bildern zur Gewebedifferenzierung im Beobachtungsareal.

Das erfindungsgemäße System für die Fluoreszenzdiagnose weist die gleichen Vorteile und Ausgestaltungen auf, wie sie in den abhängigen Patentansprüchen zur Lichtquelle angegeben sind.

Das System weist eine Steuereinrichtung zum Umschalten der Lichtquelle zwischen einem Weißlichtmodus und einem Fluoreszenzmodus auf, wobei die Steuervorrichtung dazu ausgelegt ist, in dem Fluoreszenzmodus die Intensität des von der ersten Lichtemissionseinheit emittierten ersten Lichtes mittels der Helligkeitssteuerung zu verringern und das Spektralfilter und das Neutraldichtefilter in den Beleuchtungsstrahlengang einzubringen.

Durch diese Maßnahme wird eine automatische Steuerung der Lichtquelle bereitgestellt, wobei die Steuerung auch eine automatisierte Bewegung des Spektralfilters und des Neutraldichtefilters in und aus dem Beleuchtungsstrahlengang umfasst. Die Betätigung der Steuereinrichtung kann beispielsweise über einen Fußschalter oder über einen Schalter am Kopf der Kamera realisiert sein.

Die Kamera kann eine Ein-Cip-, Zwei-Chip oder Drei-Chip-Kamera sein.

Vorzugsweise wird die Hintergrundausleuchtung mit einer Intensität im Beobachtungsareal bereitgestellt, die 0,01% bis 0,2%, vorzugsweise von 0,05% bis 0,1% der maximalen Ausgangsintensität der ersten Lichtemissionseinheit beträgt.

Für eine Weißlichtbeobachtung des Beobachtungsareals wird vorzugsweise das gesamte erste Wellenlängenspektrum des ersten Lichtes, vorzugsweise ohne Intensitätsreduktion, in das Beobachtungsareal übertragen.

Weitere Vorteile und Merkmale ergeben sich aus der nachfolgenden Beschreibung der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschreiben. Es zeigen:
- Fig. 1: ein Ausführungsbeispiel eines Systems für die Fluoreszenzdiagnose in einem Blockschaltbild; und
- Fig. 2: ein Ausführungsbeispiel einer Lichtquelle des Systems in Fig. 1.

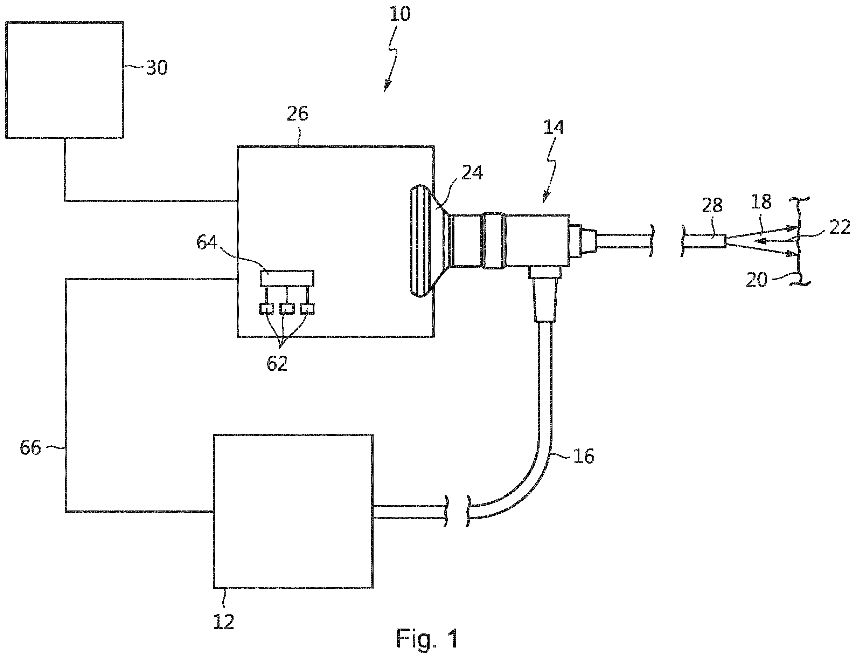

In Fig. 1 ist ein mit dem allgemeinen Bezugszeichen 10 versehenes System für die Fluoreszenzdiagnose gezeigt. Im gezeigten Ausführungsbeispiel ist das System 10 ein endoskopisches Fluoreszenzdiagnosesystem.

Das System 10 weist eine Lichtquelle 12 auf, die in einem Fluoreszenzmodus Licht in einem schmalbandigen Wellenlängenspektrum zur Anregung einer Fluoreszenz emittiert, wie nachfolgend noch beschrieben wird. Die Lichtquelle 12 ist weiterhin dazu ausgelegt, Licht in einem breitbandigen Wellenlängenspektrum zu emittieren. In einem Weißlichtmodus emittiert die Lichtquelle 12 Licht mit dem vollen breitbandigen Wellenlängenspektrum. Das breitbandige Wellenlängenspektrum umfasst vorzugsweise das gesamte sichtbare Spektrum. Im Fluoreszenzmodus emittiert die Lichtquelle 12 zusätzlich zu dem schmalbandigen Wellenlängenspektrum zur Anregung der Fluoreszenz Licht für eine Hintergrundausleuchtung des Beobachtungsareals, in dem die Fluoreszenz angeregt wird. Für die Hintergrundausleuchtung wird das Licht mit dem breitbandigen Wellenlängenspektrum spektral eingeschränkt und in der Intensität stark abgeschwächt, wie später noch beschrieben wird.

Das System 10 weist ein Endoskop 14 auf, das mit der Lichtquelle 12 über einen Lichtleiter in Form eines Lichtleitkabels 16 verbunden ist. Von der Lichtquelle 12 emittiertes Licht wird über das Lichtleitkabel 16 in das Endoskop 14 eingespeist und von diesem, wie mit einem Lichtkegel 18 angedeutet, auf ein Beobachtungsareal 20, beispielsweise ein Gewebeareal im menschlichen oder tierischen Körper, gerichtet.

Das von der Lichtquelle 12 emittierte Licht im schmalbandigen Wellenlängenspektrum dient zur Anregung einer Fluoreszenz im Beobachtungsareal 20. Im Beobachtungsareal 20 kann zu diesem Zweck ein Fluoreszenzfarbstoff angereichert sein. Das schmalbandige Wellenlängenspektrum, das von der Lichtquelle 12 emittiert wird, ist der Spektralbereich der Fluoreszenzanregung des Fluoreszenzfarbstoffes. Der Fluoreszenzfarbstoff kann beispielsweise ICG (Indocyanin-Grün) sein, mit einem Absorptionspeak bei etwa 785 nm.

Das System 10 kann jedoch auch für die Autofluoreszenzdiagnose verwendet werden. In diesem Fall wird kein zusätzlicher Fluoreszenzfarbstoff in den Körper eingebracht, sondern es werden körpereigene Substanzen mittels des von der Lichtquelle 12 emittierten schmalbandigen Anregungslichtes zur Fluoreszenz angeregt. Der Begriff Fluoreszenz umfasst daher in der vorliegenden Offenbarung auch die Autofluoreszenz.

Die im Beobachtungsareal 20 angeregte Fluoreszenz, wie mit einem Pfeil 22 angedeutet ist, wird von dem Endoskop 14 empfangen und durch das (nicht gezeigte) optische System des Endoskops 14, das durch Linsen oder eine Faseroptik gebildet sein kann, zu einem Okular 24 geführt, an dem eine Kamera 26 angeschlossen ist. Die Kamera 26 kann einen einzelnen Bildaufnehmerchip, oder zwei oder drei Bildaufnehmerchips aufweisen. Aus dem Beobachtungsareal rückgestreutes Licht der Hintergrundausleuchtung wird ebenfalls von dem Endoskop 14 empfangen und durch das optische System des Endoskops 14 zur Kamera 26 geführt.

Es versteht sich, dass die Kamera 26 auch in das Endoskop 14 integriert sein kann, wobei heutzutage miniaturisierte Kameras erhältlich sind, die sogar in die Spitze 28 des Endoskops 14 integriert werden können.

Des Weiteren versteht es sich, dass das System 10 anstelle des Endoskops 14 ein Mikroskop oder ein Exoskop aufweisen kann.

Die Kamera 26 ist mit einem Monitor 30 verbunden, auf dem das Kamerabild visuell dargestellt werden kann.

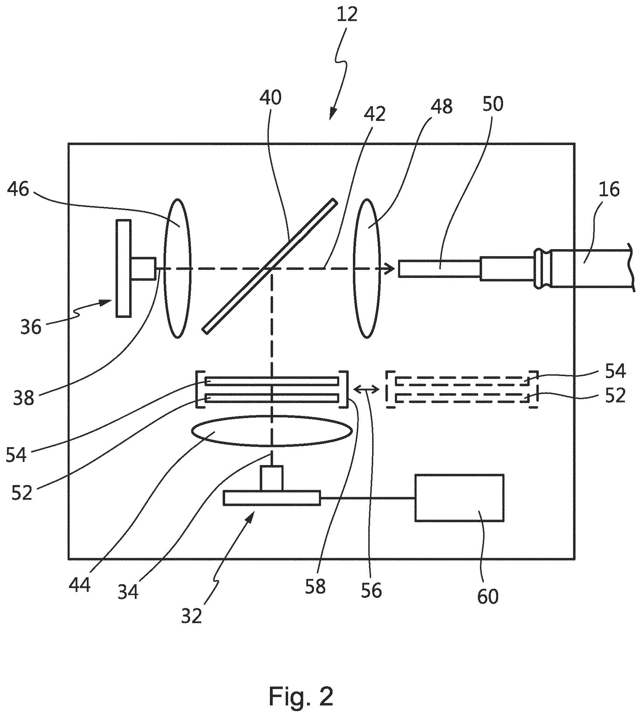

Mit Bezug auf Fig. 2 wird nachfolgend die Lichtquelle 12 näher beschrieben.

Die Lichtquelle 12 stellt einen Beleuchtungsstrahlengang bereit. Die Lichtquelle 12 weist eine erste Halbleiterleuchtmittel-basierte Lichtemissionseinheit 32 auf. Die Lichtemissionseinheit 32 ist dazu ausgelegt, erstes Licht 34 (das Bezugszeichen 34 wird nachfolgend auch für den Beleuchtungsstrahlengang der ersten Lichtemissionseinheit 32 verwendet), das durch eine unterbrochene Linie angedeutet ist, in einem breitbandigen ersten Wellenlängenspektrum zu emittieren. Die Lichtemissionseinheit 32 ist insbesondere dazu ausgelegt, Weißlicht zu emittieren. Die Lichtemissionseinheit 32 kann beispielsweise eine Weißlicht-Leuchtdiode (Weißlicht-LED) sein. Die Weißlicht-LED kann insbesondere eine Phosphor-konvertierte Weißlicht-LED sein. Phosphor wird üblicherweise als Leuchtstoff für eine blaue LED verwendet, um Weißlicht zu erzeugen. Anstelle von Phosphor kann die LED jedoch auch einen anderen Leuchtstoff aufweisen, mit dem Weißlicht erzeugt werden kann.

Die Lichtquelle 12 weist eine zweite Halbleiterleuchtmittel-basierte Lichtemissionseinheit 36 auf, die dazu ausgelegt ist, zweites Licht 38 (das Bezugszeichen 38 wird nachfolgend auch für den Beleuchtungsstrahlengang der zweiten Lichtemissionseinheit 36 verwendet), das durch eine unterbrochene Linie angedeutet ist, in einem schmalbandigen zweiten Wellenlängenspektrum zur Anregung von Fluoreszenz zu emittieren. Die zweite Lichtemissionseinheit 36 kann eine schmalbandig emittierende Leuchtdiode sein, die im blauen, grünen, roten, nah-infraroten oder infraroten Wellenlängenspektrum emittiert, je nach zur Fluoreszenz anzuregendem Fluoreszenzfarbstoff. Die zweite Lichtemissionseinheit 36 kann beispielsweise eine Leuchtdiode aufweisen, deren Emissionspeak bei etwa 785 nm liegt, so dass mit einer solchen Leuchtdiode beispielsweise die Fluoreszenz von ICG angeregt werden kann. Im Falle der Anregung von Autofluoreszenz kann die zweite Lichtemissionseinheit 36 eine Leuchtdiode aufweisen, die schmalbandig mit einem Emissionspeak bei etwa 405 nm Licht emittiert. Anstelle einer Leuchtdiode kann die zweite Lichtemissionseinheit 36 auch eine Laserdiode aufweisen, die bspw. im nah-infraroten Spektralbereich oder infraroten Spektralbereich schmalbandig emittiert.

Die Lichtquelle 12 weist weiterhin einen Strahlkombinierer (bzw. Strahlteiler) 40 auf, der das von der ersten Lichtemissionseinheit 32 emittierte Licht 34 und das von der zweiten Lichtemissionseinheit 36 emittierte Licht 38 zu einem gemeinsamen Beleuchtungsstrahlengang 42 kombiniert. Der Strahlkombinierer 40 kann als dichroitischer Spiegel ausgebildet sein.

Der ersten Lichtemissionseinheit 32 kann eine Optik 44 zugeordnet sein, die das von der Lichtemissionseinheit 32 emittierte Licht kollimiert. Ebenso kann der zweiten Lichtemissionseinheit 36 eine Optik 46 zugeordnet sein, die das von der Lichtemissionseinheit 36 emittierte Licht kollimiert. Eine weitere Optik 48 kann dem Strahlkombinierer 40 nachgeordnet sein, um das von der Lichtemissionseinheit 32 und/oder der Lichtemissionseinheit 36 emittierte Licht zu bündeln, um es möglichst verlustfrei in ein Faserbündel 50 des Lichtleitkabels 16 einzukoppeln. Die Optiken 44, 46 und 48 können auch asphärisiert sein.

Der ersten Lichtemissionseinheit 32 sind ein Spektralfilter 52 und ein optisches Intensitätsabschwächungselement 54 zugeordnet. Das Spektralfilter 52 und das optische Intensitätsabschwächungselement 54, die als optische Einheit ausgebildet sein können, werden im Fluoreszenzmodus des Systems 10 bzw. der Lichtquelle 12 für die Hintergrundausleuchtung des Beobachtungsareals 20 zusammen verwendet. Entgegen der in Fig. 2 dargestellten Reihenfolge des Spektralfilters 52 und des Intensitätsabschwächungselements 54 kann die Reihenfolge auch vertauscht sein. Das Intensitätsabschwächungselement 54 weist in dem vorliegenden Ausführungsbeispiel ein oder mehrere Neutraldichtefilter auf.

Das Spektralfilter 52 ist dazu ausgelegt, Spektralanteile des Wellenlängenspektrums des ersten Lichtes 34, die dem Farbkanal der Kamera 26 zugeordnet sind, mit dem die Fluoreszenz zu detektieren ist, zu blockieren, und übrige Spektralanteile des breitbandigen Wellenlängenspektrums des ersten Lichtes 34 durchzulassen. Wenn beispielsweise die Fluoreszenz im Blau-Kanal der Kamera 26 detektiert wird, ist das Spektralfilter 52 so ausgelegt, dass es die blauen Spektralanteile im breitbandigen Wellenlängenspektrum des ersten Lichtes 34 blockiert, und die roten und grünen Spektralanteile des Lichts 34 für die Hintergrundausleuchtung durchlässt. In diesem Beispiel kann das Spektralfilter 52 als Langpassfilter ausgebildet sein. Wenn in einem anderen Beispiel die Fluoreszenz im Rot-Kanal der Kamera 26 detektiert wird, ist das Spektralfilter 52 so ausgelegt, dass es die roten Spektralanteile des Lichts 34 blockiert, und die grünen und blauen Spektralanteile des Lichts 34 durchlässt. In diesem Fall kann das Spektralfilter 52 als Kurzpassfilter ausgebildet sein. Wenn in einem weiteren Beispiel die Fluoreszenz im Grün-Kanal der Kamera 26 detektiert wird, kann das Spektralfilter 52 als Bandsperrfilter ausgebildet sein, so dass es die grünen Spektralanteile des breitbandigen Wellenlängenspektrums des ersten Lichts 34 blockiert und die roten und blauen Spektralanteile des Lichts 34 durchlässt.

Das Spektralfilter 52 dient somit zur spektralen Einengung des breitbandigen Wellenlängenspektrums des ersten Lichts 34 für die Hintergrundausleuchtung im Fluoreszenzmodus.

Das Neutraldichtefilter 54 dient dazu, die Intensität des von der Lichtemissionseinheit 32 emittierten Lichtes 34 stark zu verringern, damit die Hintergrundausleuchtung die Fluoreszenz im Bild der Kamera 26 nicht überstrahlt. Das Neutraldichtefilter 54, das auch als Graufilter bezeichnet werden kann, schwächt die Intensität des Lichtes 34 breitbandig ab. Das Neutraldichtefilter 54 kann eine Neutraldichte in einem Bereich von 1 bis 4, vorzugsweise in einem Bereich von 1,5 bis 3, weiter vorzugsweise eine Neutraldichte von 2 aufweisen. Eine Neutraldichte von 1 bedeutet, dass das Neutraldichtefilter 54 10% des auf das Neutraldichtefilter 54 einfallenden Lichtes durchlässt. Eine Neutraldichte von 2 bedeutet, dass das Neutraldichtefilter 54 1% des auf es einfallenden Lichtes 34 durchlässt. Eine Neutraldichte von 3 bedeutet eine Durchlässigkeit von 0,1%, und eine Neutraldichte von 4 bedeutet eine Durchlässigkeit von 0,01% des Neutraldichtefilters 54.

Das Spektralfilter 52 und das Neutraldichtefilter 54 sind beide beweglich, um in den Beleuchtungsstrahlengang 34 der ersten Lichtemissionseinheit 32 eingebracht und aus diesem entfernt werden zu können, wie in Fig. 2 mit einem Doppelpfeil 56 angedeutet ist. Mit unterbrochenen Linien sind das Spektralfilter 52 und das Neutraldichtefilter 54 in Fig. 2 in ihrer aus dem Beleuchtungsstrahlengang 34 der ersten Lichtemissionseinheit 32 entfernten Stellung gezeigt.

Das Neutraldichtefilter 54 und das Spektralfilter 52 können an einem gemeinsamen Filterträger 58 in Richtung des Beleuchtungsstrahlengangs 34 der Lichtemissionseinheit 32 hinter einander angeordnet gehalten sein, der über einen (nicht gezeigten) Stellantrieb verfügt, um den Filterträger 58 gemäß einem Doppelpfeil 56 zu bewegen. Anstatt, wie in Fig. 2 gezeigt, einer translatorischen Beweglichkeit des Trägers 58 und damit der Filter 52 und 54 können diese auch an einem drehbaren Filterrad angeordnet sein, das um eine Drehachse gedreht oder geschwenkt werden kann, um die Filter 52, 54 in den und aus dem Beleuchtungsstrahlengang 34 der ersten Lichtemissionseinheit 32 zu bewegen.

Die Lichtquelle 12 weist des Weiteren zusätzlich zu dem Neutraldichtefilter 54 eine Helligkeitssteuerung 60 für die erste Lichtemissionseinheit 32 auf. Die Helligkeitssteuerung 60 ist hier als elektrische oder elektronische Helligkeitssteuerung ausgebildet, die eine Dimmschaltung aufweist, mittels der der Treiberstrom für die Lichtemissionseinheit 32 reduziert werden kann, um so die Intensität des emittierten ersten Lichtes 34 zu dimmen. Die Kombination aus der Helligkeitssteuerung 60 und dem Neutraldichtefilter 54 ist besonders vorteilhaft. Halbleiterleuchtmittel, wie sie für die erste Lichtemissionseinheit 32 verwendet werden, lassen sich zwar dimmen, jedoch benötigen sie stets einen Mindest-Treiberstrom, d.h. einen Treiberstrom über einem gewissen Schwellwert. Sinkt der Treiberstrom unter den Schwellwert ab, erlischt das Halbleiterleuchtmittel. Mit einer Helligkeitssteuerung bzw. Dimmschaltung 60 lässt sich die Intensität des ersten Lichtes 34 daher nur auf ein Minimum reduzieren, das für eine geeignete Hintergrundausleuchtung im Beobachtungsareal 20 noch wesentlich zu hoch ist. Das Neutraldichtefilter 54 ermöglicht es nun, die Intensität des von der Lichtemissionseinheit 32 emittierten Lichtes 34 unter die mittels der Helligkeitssteuerung bzw. Dimmschaltung 60 ohne das Neutraldichtefilter 54 erreichbaren minimalen Intensität zu reduzieren. Andererseits ermöglicht die elektrische Helligkeitssteuerung bzw. Dimmschaltung 60 eine kontinuierliche Feineinstellung der Intensität der Hintergrundausleuchtung im Beobachtungsareal 20. Über die Helligkeitssteuerung 60 kann die Intensität beispielsweise in einem Bereich von wenigen ± % variiert werden. Wenn beispielsweise die Hintergrundausleuchtung für die Gewebedifferenzierung zu gering ist, kann durch Erhöhen des Treiberstroms mittels der elektrischen Helligkeitssteuerung 60 die Intensität erhöht werden, bis die gewünschte Intensität der Hintergrundausleuchtung erreicht wird. Ebenso kann durch Erniedrigen des Treiberstroms mittels der Helligkeitssteuerung 60 die Intensität reduziert werden, sofern der Schwellwert für die Lichtemission nicht unterschritten wird. Das Neutraldichtefilter 54 kann daher so ausgelegt werden, dass es bei einem mittleren Treiberstrom für die Lichtemissionseinheit 32 einen Intensitäts-Grundwert für die Hintergrundausleuchtung bereitstellt, und über die Helligkeitssteuerung 60 kann dann der Intensitäts-Grundwert nach oben oder unten feineingestellt werden.

Die Helligkeitssteuerung 60 ist dazu ausgelegt, die Intensität des von der ersten Lichtemissionseinheit 32 emittierten ersten Lichtes auf einen Wert im Bereich von 2% bis 15%, vorzugsweise von 5% bis 10% der (maximalen) Ausgangsintensität der ersten Lichtemissionseinheit zu reduzieren. Das Neutraldichtefilter 54 ist wiederum dazu ausgelegt, die bereits durch die Helligkeitssteuerung 60 reduzierte Lichtemission weiterhin zu reduzieren, beispielsweise um einen Faktor 10 bis 1000, vorzugsweise um einen Faktor 100 (bei einem Neutraldichtefilter entspricht dies der Dichte 2).

Die Kamera 26 kann einen Bildaufnehmerchip mit drei Farbkanälen RGB (Rot-Grün-Blau) und ggf. einem weiteren Kanal W (Weiß) aufweisen. Die Kamera 26 kann jedoch auch zwei Bildaufnehmerchips aufweisen, die jeweils einen, drei oder vier Farbkanäle aufweisen. Die Kamera 26 kann jedoch auch drei Bildaufnehmerchips aufweisen, die jeweils selektiv einen Farbkanal (Rot-Grün-Blau) detektieren.

Für das System 10 sind verschiedene Betriebsarten möglich, um das System 10 in den Weißlichtmodus und den Fluoreszenzmodus zu schalten. Das Umschalten zwischen den verschiedenen Betriebsmodi kann über Schalter 62 beispielsweise am Kopf der Kamera 26 realisiert sein. Eine Steuereinrichtung 64, die hier beispielhaft am Kopf der Kamera 26 gezeigt ist, aber auch in die Lichtquelle 12 integriert sein kann, steht über eine Signalleitung 66 mit der Lichtquelle 12 in Verbindung. Die Steuereinrichtung 64 schaltet die Lichtquelle 12 je nach der gewählten Betriebsart zwischen dem Weißlichtmodus und dem Fluoreszenzmodus um. Der Weißlichtmodus und der Fluoreszenzmodus können beispielsweise einzeln ein- und ausgeschaltet werden. Es besteht auch eine Möglichkeit, eine Umschaltung zwischen dem Weißlichtmodus und dem Fluoreszenzmodus geshuttert zu realisieren, beispielsweise synchron mit der Kamera mit einer Frequenz von 50 bis 60 Hz. Es können auch beide Lichtemissionseinheiten 32 und 36 gleichzeitig betrieben werden, wobei in diesem Fall die Kamera 26 eine Zwei-Chip-Kamera 26 sein sollte.

Im Weißlichtmodus wird die Lichtquelle 12 so angesteuert, dass nur die erste Lichtemissionseinheit 32 Licht, und zwar Weißlicht emittiert, wobei das Weißlicht ohne spektrale Einengung in das Beobachtungsareal 20 übertragen wird. Das Spektralfilter 52 und 54 befindet sich dabei nicht im Beleuchtungsstrahlengang der Lichtemissionseinheit 32, sondern ist aus diesem heraus verfahren (unterbrochene Linien in Fig. 2). Im Weißlichtmodus kann des Weiteren die elektrische oder elektronische Helligkeitssteuerung 60 die Lichtemissionseinheit 32 für eine besonders helle Weißlicht-Ausleuchtung des Beobachtungsareals 20 mit maximalem Treiberstrom versorgen.

Beim Umschalten vom Weißlichtmodus in den Fluoreszenzmodus ist die Steuereinrichtung 64 dazu ausgelegt, die Intensität des von der ersten Lichtemissionseinheit 32 emittierten ersten Lichtes 34 mittels der elektrischen oder elektronischen Helligkeitssteuerung (Dimmschaltung) 60 zu verringern und das Spektralfilter 52 und das Neutraldichtefilter 54 in den Beleuchtungsstrahlengang des ersten Lichtes 34 einzubringen. Gleichzeitig aktiviert die Steuereinrichtung 64 die zweite Lichtemissionseinheit 36, die das Fluoreszenzanregungslicht emittiert. Mittels der durch die erste Lichtemissionseinheit 32 bereitgestellten Hintergrundausleuchtung kann dann die Fluoreszenz im Beobachtungsareal 20 mit verbesserter Orientierung des Betrachters im Beobachtungsareal 20 auf dem Monitor 30 beobachtet werden. Die erste Lichtemissionseinheit 32 dient somit im Weißlichtmodus für die Weißlichtbeobachtung, wobei das von der Lichtemissionseinheit 32 emittierte Licht 34 dann weder spektral eingeengt noch in seiner Intensität reduziert ist, und im Fluoreszenzmodus dient die erste Lichtemissionseinheit 32 zur Bereitstellung von Hintergrundausleuchtung, die spektral eingeengt und in ihrer Intensität stark reduziert ist.

Ein Verfahren zur Fluoreszenzdiagnose kann wie folgt durchgeführt werden. Das erste Licht 34 wird mit einem breitbandigen ersten Wellenlängenspektrum mittels der Lichtemissionseinheit 32 emittiert. Gleichzeitig wird das zweite Licht 38 in einem schmalbandigen zweiten Wellenlängenspektrum von der zweiten Lichtemissionseinheit 36 emittiert. Mittels der Kamera 26 wird die durch das von der zweiten Lichtemissionseinheit 36 emittierte Licht angeregte Fluoreszenz im Beobachtungsareal 20 detektiert bzw. beobachtet. Mittels des Spektralfilters 52 werden dabei Spektralanteile des von der ersten Lichtemissionseinheit 32 emittierten ersten Wellenlängenspektrums, die dem Farbkanal der Kamera 26 zugeordnet sind, mit der die Fluoreszenz zu detektieren ist, geblockt, und die übrigen Spektralanteile des ersten Lichtes 34 werden in das Beobachtungsareal 20 zum Bereitstellen einer Hintergrundausleuchtung durchgelassen. Das Bereitstellen der Hintergrundausleuchtung umfasst außerdem ein Dimmen der Intensität des emittierten ersten Lichtes 34 mittels der elektrischen oder elektronischen Helligkeitssteuerung 60 für die erste Lichtemissionseinheit 32, und ein weiteres Reduzieren der Intensität des emittierten ersten Lichtes 34 unter die mittels der elektrischen Helligkeitssteuerung 60 erreichbare minimale Intensität mit Hilfe des Neutraldichtefilters 54. Hintergrundbilder des Beobachtungsareals (Bereiche, in denen keine Fluoreszenz auftritt) werden in anderen Farbkanälen der Kamera 26 als dem Farbkanal, in dem die Fluoreszenz detektiert wird, basierend auf der Hintergrundausleuchtung aufgenommen und können zusammen mit der Fluoreszenz auf dem Monitor 30 beobachtet werden.

Vorzugsweise wird die Hintergrundausleuchtung mit einer Intensität im Beobachtungsareal 20 bereitgestellt, die 0,01% bis 0,2%, vorzugsweise von 0,05% bis 0,1% der Ausgangsintensität (maximale Intensität) der ersten Lichtemissionseinheit 32 beträgt.

Für eine Weißlichtbeobachtung des Beobachtungsareals 20 wird das gesamte erste Wellenlängenspektrum des ersten Lichtes 34 ohne Intensitätsreduktion in das Beobachtungsareal 20 übertragen.

## Patentansprüche

1. Lichtquelle für die Fluoreszenzdiagnose, wobei die Lichtquelle (12) einen Beleuchtungsstrahlengang bereitstellt, mit einer ersten Halbleiterleuchtmittel-basierten Lichtemissionseinheit (32), die dazu ausgelegt ist, erstes Licht (34) in einem breitbandigen ersten Wellenlängenspektrum zu emittieren, und mit einer zweiten Halbleiterleuchtmittel-basierten Lichtemissionseinheit (36), die dazu ausgelegt ist, zweites Licht (38) in einem schmalbandigen zweiten Wellenlängenspektrum zur Anregung einer Fluoreszenz zu emittieren, weiterhin mit einem Spektralfilter (52) für die erste Lichtemissionseinheit (32), das dazu ausgelegt ist, Spektralanteile des ersten Wellenlängenspektrums, die einem Farbkanal einer Kamera (26) zugeordnet sind, mit dem die Fluoreszenz zu detektieren ist, zu blockieren, und übrige Spektralanteile des ersten Wellenlängenspektrums durchzulassen, weiterhin mit einer Helligkeitssteuerung (60) für die erste Lichtemissionseinheit (32) zum Dimmen der Intensität des emittierten ersten Lichtes (34), und mit einem optischen Intensitätsabschwächungselement (54) für die erste Lichtemissionseinheit (32), mit dem die Intensität des emittierten ersten Lichtes (34) unter die mittels der Helligkeitssteuerung (60) ohne das Intensitätsabschwächungselement erreichbaren minimalen Intensität reduziert werden kann, wobei das Intensitätsabschwächungselement (54) beweglich ist, um in den Beleuchtungsstrahlengang (38) der ersten Lichtemissionseinheit (32) eingebracht zu werden und aus diesem entfernt zu werden, wobei das Spektralfilter (52) beweglich ist, um in den Beleuchtungsstrahlengang (34) der ersten Lichtemissionseinheit (32) eingebracht zu werden und aus diesem entfernt zu werden, wobei das Spektralfilter (52) und das Intensitätsabschwächungselement (54) an einem gemeinsamen Filterträger (58) in Richtung des Beleuchtungsstrahlengangs (34) hintereinander angeordnet sind, wobei der Filterträger (58) einen Stellantrieb aufweist, wobei der Stellantrieb mit einer Steuereinrichtung (64) verbunden ist, die beim Umschalten der Lichtquelle zwischen Weißlichtmodus und Fluoreszenzmodus den Stellantrieb aktiviert, um das Spektralfilter und das Intensitätsabschwächungselement im Weißlichtmodus aus dem Beleuchtungsstrahlengang der ersten Lichtemissionseinheit (32) zu entfernen und im Fluoreszenzmodus in diesen einzubringen.

2. Lichtquelle nach Anspruch 1, wobei die erste Lichtemissionseinheit (32) eine Weißlicht-Leuchtdiode aufweist, und/oder wobei die zweite Lichtemissionseinheit (36) eine schmalbandig emittierende Leuchtdiode oder eine Laserdiode aufweist.

3. Lichtquelle nach Anspruch 1 oder 2, wobei die Helligkeitssteuerung eine elektrische oder elektronische Dimmschaltung für die erste Lichtemissionseinheit (32) aufweist.

4. Lichtquelle nach einem der Ansprüche 1 bis 3, wobei die Helligkeitssteuerung (60) dazu ausgelegt ist, die Intensität des von der ersten Lichtemissionseinheit (32) emittierten ersten Lichtes (34) auf einen Wert im Bereich von 2% bis 15%, vorzugsweise von 5% bis 10% der maximalen Ausgangsintensität der ersten Lichtemissionseinheit (32) zu reduzieren.

5. Lichtquelle nach einem der Ansprüche 1 bis **4,** wobei das Intensitätsabschwächungselement (54) dazu ausgelegt ist, die mit der Helligkeitssteuerung eingestellte Intensität des emittierten ersten Lichtes (34) um einen Faktor im Bereich von 10 bis 1000, vorzugsweise von 10 bis 200, weiter vorzugsweise von 50 bis 150 zu verringern.

6. Lichtquelle nach einem der Ansprüche 1 bis 5, wobei das Intensitätsabschwächungselement (54) ein Neutraldichtedichtefilter aufweist, insbesondere wobei das Neutraldichtefilter (54) eine Neutraldichte in einem Bereich von 1 bis **4,** vorzugsweise in einem Bereich von 1,5 bis 3, weiter vorzugsweise eine Neutraldichte von 2 aufweist.

7. Lichtquelle nach einem der Ansprüche 1 bis 6, wobei das Spektralfilter (52) ein Langpassfilter, ein Bandsperrfilter oder ein Kurzpassfilter ist.

8. System für die Fluoreszenzdiagnose, mit einer Lichtquelle (12) nach einem der Ansprüche 1 bis 7, und mit einer Kamera (26) mit einem ersten Farbkanal zur Detektion von Fluoreszenz in einem Beobachtungsareal (20) und weiteren Farbkanälen zur Aufnahme von Hintergrundbildern zur Orientierung und Generierung eines Farbkontrastes zwischen dem ersten Kanal und den weiteren Farbkanälen, um damit eine Gewebedifferenzierung im Beobachtungsareal (20) zu ermöglichen.

9. System nach Anspruch 8, wobei die Kamera (26) eine 1-Chip-, 2-Chip oder 3-Chip-Kamera ist.

## Claims

1. Light source for fluorescence diagnosis, the light source (12) providing an illumination beam path, and comprising a first semiconductor-illuminant based light emission unit (32) configured to emit first light (34) in a broadband first wavelength spectrum, and a second semiconductor-illuminant based light emission unit (36) configured to emit second light (38) in a narrowband second wavelength spectrum for excitation of fluorescence, further comprising a spectral filter (52) for the first light emission unit (32), that is configured to block spectral components of the first wavelength spectrum which are assigned to a color channel of a camera (26) the fluorescence is to be detected with, and to transmit remaining spectral components of the first wavelength spectrum, further comprising a brightness control (60) for the first light emission unit (32) for dimming the intensity of the emitted first light (34), and an optical intensity attenuator (54) for the first light emission unit (32) by which the intensity of the emitted first light (34) can be reduced below the minimum intensity obtainable through the brightness control (60) without the intensity attenuator, wherein the intensity attenuator (54) is movable in order to be introducible into the illumination beam path (38) of the first light emission unit (32) and to be removable from the illumination beam path (34), wherein the spectral filter (52) is movable in order to be introducible into the illumination beam path (34) of the first light emission unit (32) and to be removable from the illumination beam path (34), wherein the spectral filter (52) and the intensity attenuator (54) are arranged one behind the other in direction of the illumination beam path (34) on a joint filter carrier (58), wherein the filter carrier (58) comprises an actuator, wherein the actuator is connected to a control circuit (64) which actuates the actuator upon switching of the light source between white-light mode and fluorescence mode, in order to remove the spectral filter and the intensity attenuator in the white-light mode from the illumination beam path of the first light emission unit (32), and to introduce them into same in the fluorescent mode.

2. Light source of anyone of claims 1 to 4, wherein the first light emission unit (32) comprises a white-light light emitting diode, and/or wherein the second light emission unit (36) comprises a narrowband light emitting diode or a laser diode.

3. Light source of claim 1 or 2, wherein the brightness control comprises an electrical or electronic dimming circuit for the first light emission unit (32).

4. Light source of anyone of claims 1 to 3, wherein the brightness control (60) is configured to reduce the intensity of the first light (34) emitted from the first light emission unit (32) to a value in a range from 2% to 15%, preferably from 5% to 10%, of the maximum output intensity of the first light emission unit (32).

5. Light source of anyone of claims 1 to 4, wherein the intensity attenuator (54) is configured to reduce the intensity of the emitted first light (34) that is adjusted through the brightness control, by a factor in a range from 10 to 1000, preferably from 10 to 200, further preferably from 50 to 150.

6. Light source of anyone of claims 1 to 5, wherein the intensity attenuator (54) comprises a neutral density filter, in particular wherein the neutral density filter (54) comprises a neutral density in a range from 1 to 4, preferably in a range from 1.5 to 3, further preferably a neutral density of 2.

7. Light source of anyone of claims 1 to 6, wherein the spectral filter (52) is a long pass filter, a band reject filter, or a short pass filter.

8. System for fluorescence diagnosis, comprising a light source (12) of anyone of claims 1 to 7, and a camera (26) comprising a first color channel for detection of fluorescence in an observation area (20) and further color channels for picking up background images for orientation and generation of a color contrast between the first channel and the further color channels, in order to enable tissue differentiation in the observation area (20).

9. System of claim 8, wherein the camera (26) is a 1-chip-, 2-chip or 3-chip-camera.

## Revendications

1. Source de lumière pour le diagnostic par fluorescence, dans laquelle la source de lumière (12) fournit un trajet de faisceau d'éclairage, avec une première unité d'émission de lumière à base de moyens d'éclairage à semiconducteurs (32), qui est conçue pour émettre une première lumière (34) dans un premier spectre de longueur d'onde à large bande, et avec une deuxième unité d'émission de lumière (36) à base de moyens d'éclairage à semiconducteurs, qui est conçue pour émettre une deuxième lumière (38) dans un deuxième spectre de longueur d'onde à bande étroite pour exciter une fluorescence, par ailleurs avec un filtre spectral (52) pour la première unité d'émission de lumière (32), qui est conçu pour bloquer des fractions spectrales du premier spectre de longueur d'onde, qui sont associées à un canal de couleur d'une caméra (26) permettant de détecter la fluorescence et pour laisser passer des fractions spectrales restantes du premier spectre de longueur d'onde, par ailleurs avec une commande de luminosité (60) pour la première unité d'émission de lumière (32) pour atténuer l'intensité de la première lumière émise (34), et avec un élément d'affaiblissement d'intensité optique (54) pour la première unité d'émission de lumière (32) permettant de réduire l'intensité de la première lumière émise (34) en dessous de l'intensité minimale atteignable au moyen de la commande de luminosité (60) sans l'élément d'affaiblissement d'intensité, dans laquelle l'élément d'affaiblissement d'intensité (54) est mobile pour être introduit dans et retiré du trajet de faisceau d'éclairage (38) de la première unité d'émission de lumière (32), dans laquelle le filtre spectral (52) est mobile pour être introduit dans et retiré du trajet de faisceau d'éclairage (34) de la première unité d'émission de lumière (32), dans laquelle le filtre spectral (52) et l'élément d'affaiblissement d'intensité (54) sont disposés l'un après l'autre sur un support de filtre commun (58) en direction du trajet de faisceau d'éclairage (34), dans laquelle le support de filtre (58) présente un entraînement de réglage, dans laquelle l'entraînement de réglage est relié à un système de commande (64) qui, lors de la commutation de la source de lumière entre le mode lumière blanche et le mode fluorescence, active l'entraînement de réglage pour retirer le filtre spectral et l'élément d'affaiblissement d'intensité dans le mode lumière blanche du trajet du faisceau d'éclairage de la première unité d'émission de lumière (32) et l'y introduire dans le mode fluorescence.

2. Source de lumière selon la revendication 1, dans laquelle la première unité d'émission de lumière (32) présente une diode électroluminescente à lumière blanche, et/ou dans laquelle la deuxième unité d'émission de lumière (36) présente une diode électroluminescente à bande étroite ou une diode laser.

3. Source de lumière selon la revendication 1 ou 2, dans laquelle la commande de luminosité présente un circuit d'atténuation électrique ou électronique pour la première unité d'émission de lumière (32).

4. Source de lumière selon l'une quelconque des revendications 1 à 3, dans laquelle la commande de luminosité (60) est conçue pour réduire l'intensité de la première lumière (34) émise par la première unité d'émission de lumière (32) à une valeur dans la plage de 2 % à 15 %, de préférence de 5 % à 10 % de l'intensité de sortie maximale de la première unité d'émission de lumière (32).

5. Source de lumière selon l'une quelconque des revendications 1 à 4, dans laquelle l'élément d'affaiblissement d'intensité (54) est conçu pour réduire l'intensité de la première lumière émise (34) réglée avec la commande de luminosité d'un facteur dans la plage de 10 à 1000, de préférence de 10 à 200, de préférence encore de 50 à 150.

6. Source de lumière selon l'une quelconque des revendications 1 à 5, dans laquelle l'élément d'affaiblissement d'intensité (54) présente un filtre de densité neutre, en particulier dans laquelle le filtre de densité neutre (54) présente une densité neutre dans une plage de 1 à 4, de préférence dans une plage de 1,5 à 3, de préférence encore une densité neutre de 2.

7. Source de lumière selon l'une quelconque des revendications 1 à 6, dans laquelle le filtre spectral (52) est un filtre passe-bas, un filtre d'élimination de bande ou un filtre passe-haut.

8. Système de diagnostic par fluorescence, avec une source de lumière (12) selon l'une quelconque des revendications 1 à 7, et avec une caméra (26) avec un premier canal de couleur pour la détection de fluorescence dans une zone d'observation (20) et d'autres canaux de couleur pour l'enregistrement d'images de fond pour l'orientation et la génération d'un contraste de couleur entre le premier canal et les autres canaux de couleur pour permettre ainsi une différenciation tissulaire dans la zone d'observation (20).

9. Système selon la revendication 8, dans lequel la caméra (26) est une caméra à 1, 2 ou 3 puces.
